# EUROPÄISCHE PATENTSCHRIFT

(11) **EP 2 409 961 B1**
(45) Veröffentlichungstag und Bekanntmachung des Hinweises auf die Patenterteilung: **27.11.2013**
(21) Anmeldenummer: 10170332.0
(22) Anmeldetag: 21.07.2010
(51) Int. Cl.: C07C 45/74, C07C 49/203, B01J 23/10, B01J 37/08, B01J 23/92

(54) **Verfahren zur Herstellung von Pseudojonon**
Process for the preparation of pseudoionone
Procédé pour la préparation de pseudoionone

(43) Veröffentlichungstag der Anmeldung: 25.01.2012
(73) Patentinhaber: DSM IP Assets B.V., 6411 TE Heerlen (NL)
(72) Erfinder: Hölderich, Wolfgang, 4303 Kaiseraugst (CH); Ritzerfeld, Verena, 4303 Kaiseraugst (CH); Russbüldt, Bernhard, Markus, Ernst, 4303 Kaiseraugst (CH); Fleischhauer, Erhard, Henning, 4303 Kaiseraugst (CH); Bonrath, Werner, 4303 Kaiseraugst (CH); Karge, Reinhard, 4303 Kaiseraugst (CH); Schütz, Jan, 4303 Kaiseraugst (CH)
(74) Vertreter: Steck, Melanie

(56) Entgegenhaltungen:
- WO-A1-03/047748
- RUSSBUELDT B M E ET AL: "New rare earth oxide catalysts for the transesterification of triglycerides with methanol resulting in biodiesel and pure glycerol", JOURNAL OF CATALYSIS, ACADEMIC PRESS, DULUTH, MN, US, Bd. 271, Nr. 2, 4. Mai 2010 (2010-05-04), Seiten 290-304, XP027021150, ISSN: 0021-9517 [gefunden am 2010-03-20]

## Beschreibung

Die vorliegende Erfindung betrifft ein Verfahren zur Herstellung von Pseudojonon und die darin verwendeten Katalysatoren. Genauer betrifft die vorliegende Erfindung die Herstellung von Pseudojonon aus Citral (E/Z-3,7-Dimethyl-2,6-octadien-1-al) und Aceton in flüssiger Phase mit einem heterogenen Katalysator, wobei es sich bei dem Katalysator um Lanthanoxid handelt, dass auf besondere Weise behandelt bzw. hergestellt wurde. Die Erfindung betrifft auch den so hergestellten Lanthanoxid-Katalysator.

Pseudojonon (6,10-Dimethyl-3,5,9-undecatrien-2-on) besitzt Juvenilhormon-Aktivität und ist ein bedeutendes Zwischenprodukt bei der Herstellung von Vitamin A und E, Carotinoiden und Riechstoffen. Es wird vor allem durch Basenkatalysierte Aldolreaktion von Citral mit Aceton und Wasserabspaltung, d. h. Aldolkondensation, gewonnen. Vorteilhaft ist die Verwendung heterogener Katalysatoren, insbesondere auf inerten Trägern, da diese aus dem Reaktionsmedium leicht abgetrennt werden und, ggf. nach Regeneration, mehrmals wieder verwendet werden können.

In WO 2003/047747 und WO 2003/047748 (publ. 12.06.2003) werden Metalloxid-Katalysatoren auf y-Aluminiumoxid, deren Verwendung bei der Aldolkondensation von Citral und Aceton zu Pseudojonon, sowie spezielle Kolonnen zur Durchführung der Reaktion durch Reaktivdestillation beschrieben. Bei den Metalloxiden handelt es sich um Oxide der Elemente mit den Ordnungszahlen 39 (Y) und 57 (La) -71 (Lu), wobei Yttriumoxid und Praseodymoxid als bevorzugt hervorgehoben sind. Bei Verwendung eines Katalysators aus 5 % Pr auf y-AI₂0₃ wurde im 24-Stunden-Betrieb Pseudojonon mit einer Ausbeute von 66.7 % und einer Selektivität von 97.3 % bezogen auf Citral erhalten. Die Konzentration der Aktivkomponente bezogen auf das Gesamtgewicht des geträgerten Katalysators liegt im Bereich von 5 - 12 Gew.-%, vorzugsweise 7.5 - 10 Gew.-%. Während sich als Ergebnis umfangreicher Screening-Untersuchungen y-AI₂0₃ als besonders geeignetes Trägermaterial, was die Aktivität des Katalysators betrifft, herausstellte, wird betont, dass sowohl die Geometrie des Katalysatorträgers als auch die Einhaltung der oben genannten Konzentrationen von entscheidender Bedeutung für den Erhalt guter Raumzeitausbeuten sind.

Zur Herstellung des geträgerten Pr-Katalysators wurde der Träger mit einer Praseodymnitrat-Stammlösung aus 1170 g Praseodymoxid, 2276 g 65 %iger Salpetersäure und 1557 g Wasser, die je nach Flüssigkeitsaufnahme des Trägers mit voll entionisiertem (VE) Wasser verdünnt wurde, getränkt. Es wurde im Trockenschrank bei 120 °C getrocknet und anschliessend 2 Stunden bei 450 °C kalziniert.

DE 102 38 140 A1 (publ. 26.02.2004) beschreibt ein Verfahren und eine Apparatur zur kontinuierlichen Herstellung von längerkettigen Carbonylverbindungen am Beispiel der Herstellung von Pseudojonon aus Citral und Aceton unter Verwendung eines heterogenen Katalysators aus 5 % Pr auf y-AI₂0₃ (hergestellt durch Tränkung von y-AI₂0₃ mit einer wässrigen Lösung von Pr-Nitrat und ausschliessende Kalzinierung). Es wird eine Ausbeute von 82.6 % und eine Selektivität von 97.2 % bezogen auf Citral erreicht. Als bevorzugte Katalysatoren werden einerseits Verbindungen enthaltend Elemente der Lanthanoxid-Reihe, insbesondere La, Ce und/oder Pr, in einer Konzentration von 0.1 - 20 Gew.-%, vorzugsweise 1 - 10 Gew.-%, auf Aluminiumoxid genannt.

Um die vorstehend genannten Nachteile, d. h. die genaue Einhaltung der Verhältnisse bei der Herstellung der geträgerten Katalysatoren, und darüber hinaus die Bildung von Oxid-Mischphasen bei der Kalzinierung, die die Katalysatoraktivität, insbesondere bei Anwendung höherer Temperaturen, herabsetzen, zu vermeiden, bestand die Aufgabe, nicht geträgerte heterogene Katalysatoren aus der Lanthanoxid-Reihe zu entwickeln, die gute Ausbeuten und hohe Selektivität bei der Aldolkondensation von Citral und Aceton zu Pseudojonon liefern.

Diese Aufgabe wurde dadurch gelöst, dass ungeträgerte Lanthanoxid-Katalysatoren gefunden wurden, die die Aldolkondensation von Citral und Aceton zu Pseudojonon in hoher Ausbeute und Selektivität katalysieren, sofern sie in bestimmter Weise hergestellt sind.

In J. Catal. 271, 290-304 (2010) beschreiben B. Russbueldt et al. reine Seltene Erden-Oxide, die als Katalysatoren für die Umesterung von Triglyceriden mit Methanol geeignet sind. Am Beispiel von La₂0₃ wird die Herstellung eines reinen Lanthanoidoxid-Katalysators beschrieben. La₂0₃ wird in 65 %iger Salpetersäure gelöst und die Lösung mit Wasser verdünnt. Durch Zugabe einer wässrigen Lösung von Oxalsäure-Dihydrat wird Lathanoxalat ausgefällt, das nach Filtration und mehrmaligem Waschen mit Wasser bei 120 °C getrocknet wird. Durch Erhitzen (Kalzinierung) auf 900 °C in einem Muffelofen während 12 Stunden und Abkühlen in einem Exsikkator wird reines La₂0₃ als weisses Pulver erhalten, das durch Röntgenstrahl-Diffraktion (XRD) charakterisiert wurde. Eine Verbesserung der Katalysator-Eigenschaften konnte erreicht werden durch Senkung der Kalzinierungstemperaturen auf 700 °C, wodurch Sintereffekte minimiert wurden.

Es stellte sich heraus, dass so hergestellte reine Lanthanoxide sich auch sehr gut als heterogene Katalysatoren für die Aldolkondensation von Citral und Aceton zu Pseudojonon eignen.

Die vorliegende Erfindung betrifft daher die Verwendung reiner Lanthanoxide, die dadurch erhalten werden, dass sauerstoffhaltige Lanthansalze bei Temperaturen von mindestens 700 °C kalziniert werden, als heterogene Katalysatoren bei der Herstellung von Pseudojonon aus Citral und Aceton sowie ein Verfahren zur Herstellung von Pseudojonon aus Citral und Aceton in flüssiger Phase in Gegenwart dieser Lanthanoxid-Katalysatoren.

Die Aldolkondensation von Citral mit Aceton zu Pseudojonon findet in flüssiger Phase in an sich bekannter Weise, allerdings in Gegenwart von reinem, trägerlosem Lathanoxid als heterogenem Katalysator statt. Da Citral als Isomerengemisch vorliegt, wird auch das Pseudojonon als Gemisch von (3E,5Z)- und (3E,5E)-6,10-Dimethylundeca-3,5,9-trien-2-on enthalten, aus dem die einzelnen Komponenten gewünschtenfalls isoliert werden können.

Die Reaktion wird zweckmässigerweise in einem Temperaturbereich von 140 - 250 °C durchgeführt, vorzugsweise in einem Bereich von 160 - 200 °C und unter Normaldruck oder Drucken von 1 - 6 bara. Bei niedrigeren Temperaturen wird die Ausbeute so gering, dass das Verfahren für eine kommerzielle Anwendung nicht mehr interessant ist. Es wird mit einem Überschuss von Aceton gearbeitet, wobei die besten Ausbeuten erreicht wurden, wenn im Bereich 10 - 2 : 1 Mol/Mol gearbeitet wird. Das Verfahren ist, wie die Beispiele belegen, für diskontinuierlichen Betrieb gedacht, doch ist eine Einrichtung für kontinuierlichen Betrieb für den Fachmann prinzipiell kein Problem.

Das Verhältnis von Katalysator: Citral liegt zweckmässigerweise im Bereich von 1 : 1000 - 1 : 10 , vorzugsweise 1 : 100 - 1 : 20 (Mol/Mol).

Der Katalysator kann mehrfach verwendet werden, wobei allerdings seine Aktivität abnimmt (Wasseraufnahme). Es sollte daher in regelmässigen Abständen, die leicht empirisch bestimmt werden können, regeneriert, d. h. bei 700 - 900 °C rekalziniert werden.

Geeignete Ausgangsverbindungen für die Herstellung des reinen Lanthanoxids, La₂0₃, sind sauerstoffhaltige Lanthansalze oder Lanthansalze, die sich in solche überführen lassen, beispielsweise Lanthanoxid, Lanthannitrat, Lanthancarbonat und Lanthanoxalat und die durch Kalzinierung bei 700 - 900 °C alles Wasser und CO₂ verlieren. Eine Aufbewahrung unter einem inerten Gas und in Gegenwart wasserbindender Chemikalien, wie z. B. in einem Exsikkator, ist daher zweckmässig.

Es können auch reine, ungeträgerte Oxide anderer Lanthanoid-Elemente, z. B. von Nd oder Pr, in der vorliegenden Aldolkondensation als Katalysatoren eingesetzt werden, allerdings mit schlechteren Ausbeuten und/oder geringerer Selektivität.

Die folgenden Beispiele illustrieren die vorliegende Erfindung im Detail.

### Verwendete Chemikalien

Citral wurde mit einer Reinheit von 95 % als Isomerengemisch aus cis (Geranial) und trans (Neral) von Fa. Acros bezogen, unbehandeltes Lanthanoxid von Fa. Fluka mit einer Reinheit von 99.98 %. Es wurde technisches Aceton verwendet, das bis zu 1 % Wasser enthalten kann. Versuche zeigten, dass dieses, gegenüber dem bei der Reaktion entstehenden Wasser, kein Einfluss auf die Versuchsergebnisse hat.

### Synthese von Lanthanoxid

Es wurden 50 g (0.15 mol) unbehandeltes Lanthanoxid (Fa. Fluka) durch Zugabe von 68.39 mL konzentrierter Salpetersäure und 1.5 L VE-Wasser in die Nitrate überführt. Hierbei wurde die Lösung gerührt und leicht erhitzt, um den Auflösevorgang zu beschleunigen. Nach Abkühlen der Lösung auf Raumtemperatur wurden unter Rühren 84.27 g (0.67 Mol, 1.5 Äquiv.) Oxalsäure, gelöst in 500 mL VE-Wasser, zugesetzt und Lanthanoxalat ausgefällt. Anschließend wurde abdekantiert und über einen Büchnertrichter filtriert. Der erhaltene weiße Feststoff wurde über Nacht im Trockenschrank bei 110°C getrocknet. Es wurden 78.43 g (0.145 Mol, 94.3 % der Theorie) Lanthanoxalat erhalten. Dann wurden 2 g des Lanthanoxalats im Röhrenofen bei 650°C 18 Stunden an Luft kalziniert. Nach Abkühlen auf 50 °C im Stickstoffstrom wurde das hergestellte, weiße Lanthanoxid in ein mit Argon befülltes Schlenkrohr überführt. Weitere 6 g Lanthanoxalat wurden 18 Stunden im Muffelofen bei 700 °C zu Lanthanoxid kalziniert, im Exsikkator abgekühlt und ebenfalls in einem Schlenkrohr unter Argon gelagert.

### Experimentelles Vorgehen bei der Aldolkondensation

Alle Versuche wurden batchweise in Autoklaven mit einem Fassungsvermögen von 75 mL durchgeführt. Diese waren versehen mit einem Druckaufnehmer und einem Ventil. Die Versuche wurden auf einer kombinierten Heiz-Rührplatte durchgeführt, die zur gleichmäßigen Wärmeverteilung auf die Autoklaven mit einer Heizmanschette versehen war. Über einen Eurothermregler mit Thermoelement wurde die jeweilige Versuchstemperatur eingestellt. Vor der Versuchsreihe wurden die Autoklaven auf Dichtigkeit überprüft. Hierzu wurden die Autoklaven mit Druckluft befüllt und in ein Wasserbad gestellt.

Es wurde eine Aceton : Citral-Mischung angesetzt (molares Verhältnis 12:1). Dann wurde in ein mit einem Rührfisch versehenen Glasinlet 1 g des jeweiligen Katalysators eingewogen und mit 30 mL der jeweiligen Citral : Aceton-Lösung aufgefüllt. Hierbei wurde das Leergewicht sowie das Gewicht des befüllten Glasinlets bestimmt. Anschließend wurde das befüllte Glasinlet in den Autoklaven gestellt und dieser verschraubt. Der Autoklav wurde mit der Heizmanschette und dem Thermoelement versehen und die Versuchstemperatur mit dem Eurothermregler eingestellt. Die Versuchsdauer betrug bei allen Versuchen 3 Stunden. Die Zeitmessung startete mit dem Erreichen der Versuchstemperatur. Nach Beendigung des Versuchs wurde der Autoklav im Eisbad gekühlt und geöffnet. Das Glasinlet wurde erneut gewogen und die Reaktionsmischung zentrifugiert. Von der nun klaren Reaktionslösung wurde eine GC-Probe mit Dodecan als Standard abgegeben. Der Katalysator wurde von Citralresten befreit, getrocknet und mithilfe einer Röntgenuntersuchung charakterisiert. Einige Katalysatoren wurden ein zweites Mal eingesetzt, um ihre Aktivität zu testen.

### Verwendete Geräte und Methoden

### Gaschromatografie (GC)

Die Analyse des Reaktionsgemisches wurde mit einer FS-FFAP-Säule (25 m; CS25178-5) in einem HP 6890 Plus Gaschromatografen durchgeführt.

### Röntgenmessung (XRD)

Die Röntgenspektren wurden mit einem Pulverdiffraktometer der Marke Siemens D 5000 gemessen. Die verwendete Anode bestand aus Kupfer.

### Oberflächenbestimmung nach Brunauer, Emmett und Teller (BET)

Die Untersuchung der Proben erfolgte mit einem Micrometics ASAP 2000. Zunächst wurden die Proben ausgeheizt und mit Helium das Totvolumen ermittelt. Dann wurde mit Stickstoff die Adsorptions-und Desorptionsisotherme aufgenommen.

### Thermogravimetrie (TG)

Die Proben wurden mithilfe eines Netzsch STA 409C mit Al₂O₃ als Referenz gemessen. Die Untersuchung startete bei RT und lief bis 1110 °C, bei einer Aufheizrate von 2.0 K/min.

Aus der Thermogravimetrie ist ersichtlich, dass eine Calcinierung über 700 °C am sinnvollsten ist, wenn eine reine Lanthanoxidphase erhalten werden soll.

### Temperatur-programmierte Desorption (TPD)

Die Messung wurde mit CO₂ als Sondenmolekül mit der TPD 1100 Apparatur der Firma Thermodefinition durchgeführt. Hierzu wurden die Proben bei 300 °C im Stickstoffstrom ausgeheizt und anschließend bei 100 °C über eine Stunde mit CO₂ beladen, um Physisorption möglichst zu vermeiden. Danach wurde auf 150-200 °C aufgeheizt und mit Inertgas physisorbiertes CO₂ entfernt. Daraufhin wurde das Temperaturprogramm gestartet und die Desorption von CO₂ über die Zeit aufgenommen. Bei einer zweiten Probe wurde die TPD-Kurve ohne Beladung aufgenommen.

### Beschreibung der Figuren

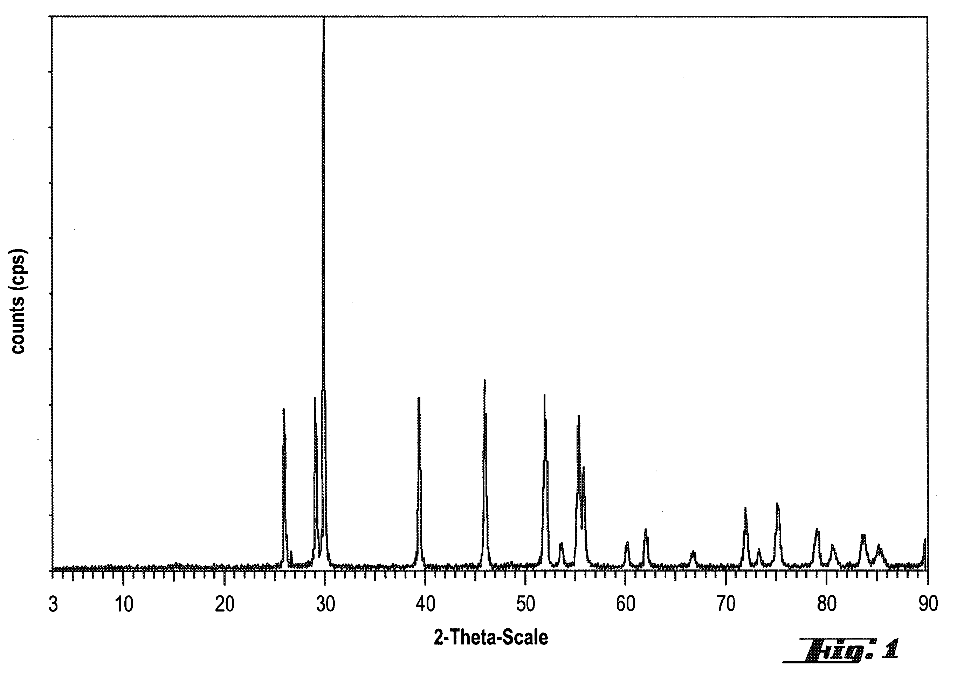

Figur 1: Röntgenspektrum von Lanthanoxid 900 °C, Katalysator C.

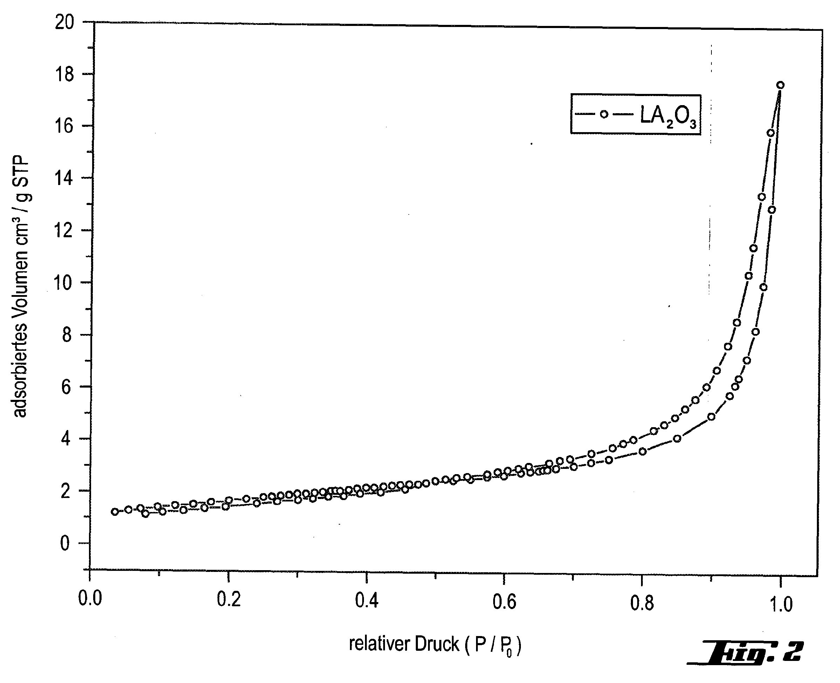

Figur 2: BET-Messung von Lanthanoxid 900°C, Katalysator **C**.
Adsorptions-/Desorptionsisotherme für N₂ von Lathanoxid 900 °C, Katalisator C

| | |
|---|---|
| BET Oberfläche: | 5.99 m²/g |
| Mikroporenfläche- und Volumen: | 0 CM³/g |

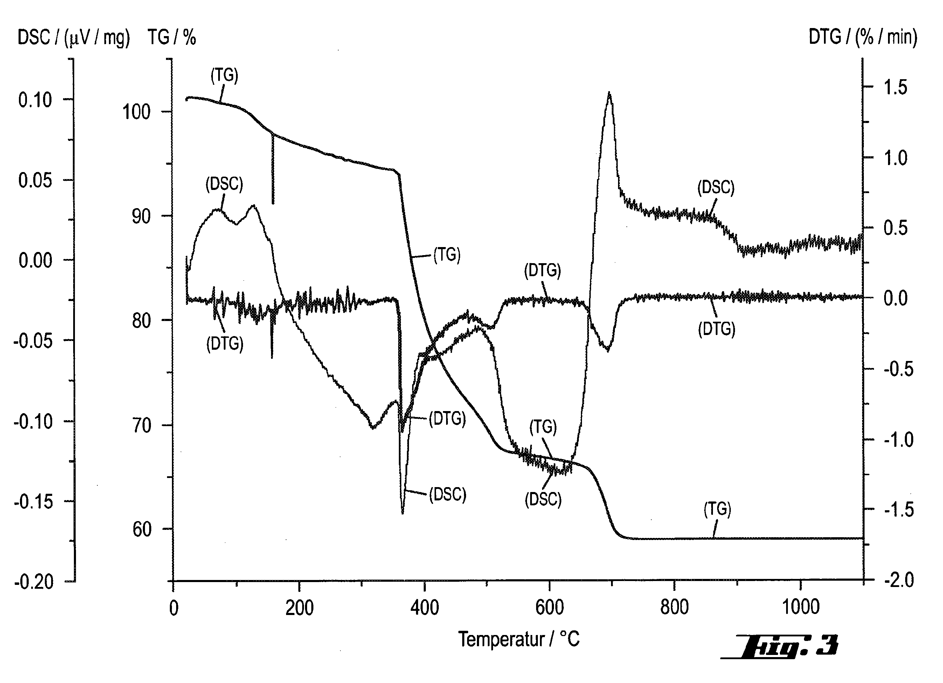

Figur 3: Thermogravimetrische Untersuchung von Lanthanoxalat.

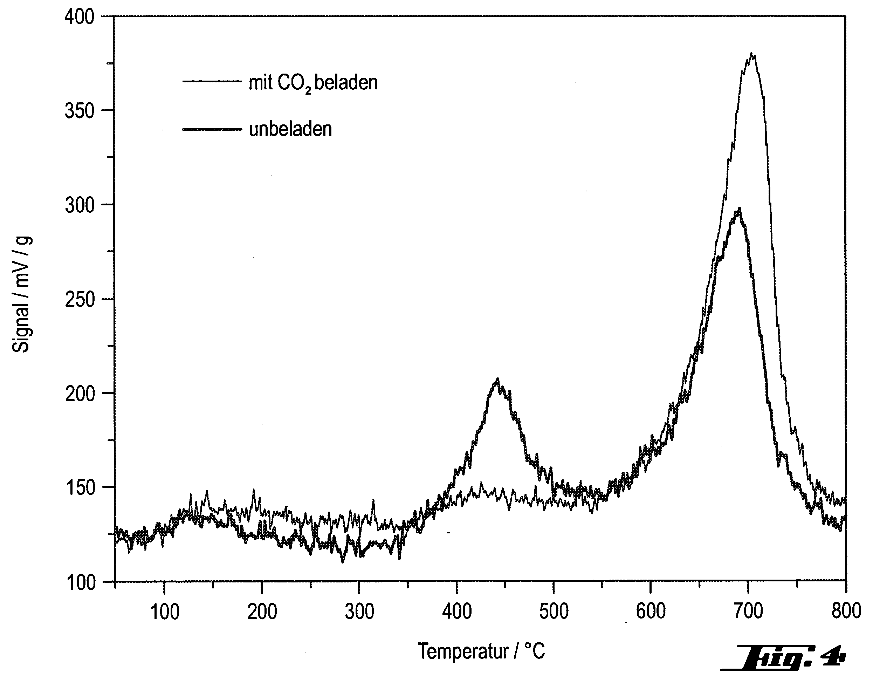

Figur 4: TPD-Untersuchung von Lanthanoxid 900°C, Katalysator **C**

Die schwach schwarze Kurve wurde nach Ausheizen der Probe auf 300 °C und anschließender Beladung mit CO₂ und die stark schwarze Kurve ohne Beladung aufgenommen. Wie zu erkennen ist, zeigen beide Kurven eine starke Desorptionsbande bei 700 °C. Darüber hinaus zeichnet sich bei der roten Kurve ein weiterer Desorptionspeak bei 450 °C ab. Dies kann einerseits bedeuten, dass die Zentren sehr stark basisch sind und daher das Sondenmolekül erst bei sehr hohen Temperaturen wieder freigeben. Andererseits ist es auch möglich, dass keine Adsorption des CO₂, sondern eine Reaktion zu einer stabilen Carbonatphase abgelaufen ist, wodurch CO₂ ebenfalls nur schwer wieder desorbieren kann: Die Ausbildung von Carbonatphasen erscheint jedoch wahrscheinlicher, da die Desorptionspeaks bei 450 °C und 700 °C mit dem Zerfall der Carbonate La₂(CO₃)O₂ und La₂(CO₃)₂O und dem damit verbundenen Gewichtsverlust in der TG übereinstimmen.

Die folgenden Beispiele demonstrieren die katalytische Wirksamkeit von Vollkatalysatoren aus reinem Lathanoxid für die Aldolreaktion von Citral und Aceton zu Pseudojonon.

### Beispiel 1

Es wurden 16,29 g La₂O₃ in 22,9 ml HNO₃ 65 % gelöst und mit Wasser auf 500 ml verdünnt. Hierzu wurde eine Lösung von 20,80 g Oxalsäure 2 H₂O in 500 ml Wasser zugegeben. Das ausgefallene Lanthanoxalat La₂(C₂O₄)₃ wurde abfiltriert, mit destilliertem Wasser gewaschen und 16 Stunden bei 120 °C getrocknet. Abschliessend wurde das La₂(C₂O₄)₃ bei drei verschiedenen Temperaturen, nämlich 650 °C (Kat **A**), 700 °C (Kat **B**) und 900°C (Kat **C**), Raumtemperatur-Zieltemperatur /12 Stunden und bei Zieltemperatur °C/12 Stunden isotherm zu reinem La₂O₃ verglüht und in einem Exsikkator über KOH abgekühlt. Mit Lanthanoxid D unbehandelt ist das kommerziell bei Fluka erhältliche, unkalzinierte Produkt gemeint.

Für die Katalyse-Versuche wurden 23.19 g Aceton, 5.01 g Citral, molares Verhältnis (Aceton : Citral) 12:1, und 1 g des reinen La₂O₃ eingesetzt. Die Reaktion wird in einem 75 ml Autoklaven mit Glaseinsatz für 3 Stunden bei 200 °C und einem autogenen Druck von 25 bar durchgeführt.

**Tabelle 1: Katalytische Wirksamkeit von reinem Lanthanoxid in der Aldolreaktion von Citral und Aceton.**

| Katalysator | La₂O₃ **A** | La₂O₃ **B** | La₂O₃ **C** | La₂O₃ **D** |
|---|---|---|---|---|
| | 650 °C | 700 °C | 900 °C | unbehandelt |
| Umsatz | 76.9 % | 78.8 % | 93.4 % | 76.2 % |
| Selektivität | 78.9 % | 76.6 % | 84.3 % | 78.7 % |
| Ausbeute | 60.7 % | 60.4 % | 78.7 % | 59.9 % |

Es konnte durch Röntgenuntersuchung gezeigt werden, dass eine Kalzinierung über 700 °C nötig ist, um die reine Oxidphase zu erhalten.

### Beispiel 2

Dieses Beispiel demonstriert die katalytische Wirksamkeit von Vollkatalysatoren aus reinem Neodymoxid oder Praseodymoxid für die Aldolreaktion.

Die Herstellung der Katalysatoren erfolgte auf dieselbe Art wie in Beispiel 1 beschrieben. Die Kalziniertemperaturen betrugen jedoch nur 650 (Kat E und H) bzw. 700 °C (Kat **F** und **I),** da eine solche Temperatur ausreicht, um die reinen Oxidphasen herzustellen. Für die Katalyse-Versuche wurden 23.19 g Aceton, 5.01 g Citral und 0,4 g des reinen Oxides eingesetzt. Die Reaktion wurde in einem 75 ml Autoklaven mit Glaseinsatz während 3 Stunden bei 200 °C durchgeführt.

Auch für die in Beispiel 2 aufgeführten Katalyse-Versuche wurden 23.19 g Aceton, 5.01 g Citral, molares Verhältnis (Aceton : Citral) 12 : 1, und 1 g des jeweilig verwendeten Katalysators eingesetzt. Die Reaktion wurde in einem 75 ml Autoklaven mit Glaseinsatz während 3 Stunden bei 200 °C und einem autogenen Druck von 25 bar durchgeführt.

**Tabelle 2: Katalytische Wirksamkeit von reinem Neodymoxid und Praseodymoxid in der Aldolreaktion von Citral und Aceton.**

| Katalysator | Nd₂O₃ **E** | Nd₂O₃ **F** | Nd₂O₃ **G** | Pr₆O₁₁ **H** | Pr₆O₁₁ , **I** | Pr₆O₁₁ **J** |
|---|---|---|---|---|---|---|
| | 650 °C | 700 °C | unbehandelt | 650 °C | 700 °C | unbehandelt |
| Umsatz | 14.1 % | 60.6 % | 53.2 % | 6.9 % | 54.7 % | 66.8 % |
| Selektivität | 64.1 % | 79.3 % | 80.3 % | 54.2 % | 77.2 % | 79.2 % |
| Ausbeute | 9.0 % | 48.1 % | 42.8 % | 3.7 % | 42.2 % | 53.0 % |

### Beispiel 3

Dieses Beispiel zeigt den Einfluss der Temperatur auf Umsatz, Selektivität und Ausbeute der Aldolreaktion. Hierbei wurden sämtliche Versuche mit 1 g La₂O₃ 900 °C, Katalysator C, einem molaren Verhältnis von 12 : 1 (Aceton : Citral) bei 200 °C und 3 Stunden Reaktionszeit durchgeführt.

**Tabelle 3:Einfluss der Temperatur auf die katalytische Aktivität.**

| Temperatur °C | Umsatz | Selektivität | Ausbeute |
|---|---|---|---|
| 60 | 0.6% | 0.0% | 0.0% |
| 80 | 0.1 % | 0.0% | 0.0% |
| 120 | 17.4% | 51.7% | 9.0% |
| 160 | 69.9 % | 87.5 % | 78.7 % |
| 200 | 93.4 % | 84.3 % | 78.7 % |

Es wurde eine scharfe Temperaturlinie bei 160 °C gefunden, bei der die Reaktion verstärkt einsetzt.

### Beispiel 4

Dieses Beispiel zeigt den Einfluss der Katalysatormenge auf die katalytische Aktivität. Hierbei wurden sämtliche Versuche mit La₂O₃ 900 °C Katalysator **C**, einem molaren Verhältnis von 12 : 1 (Aceton : Citral) bei 200 °C, einem autogenen Druck von 25 bar und 3 Stunden Reaktionszeit durchgeführt.

**Tabelle 4: Einfluss der Katalysatormenge auf die katalytische Aktivität.**

| Katalysatormenge [g] | Umsatz | Selektivität | Ausbeute |
|---|---|---|---|
| 0.4 | 53.2 % | 74.3 % | 39.6 % |
| 0.6 | 65.9 % | 72.0 % | 47.4 % |
| 0.8 | 75.5 % | 67.1 % | 50.7 % |
| 1 | 81.4 % | 64.1 % | 52.2 % |
| 2 | 84.9% | 54.2 % | 46.0% |

Wie erwartet steigt der Umsatz bei steigender Katalysatormenge. Die Selektivität sinkt jedoch.

### Beispiel 5

Dieses Beispiel zeigt die Recyclisierung des Katalysators ohne Regeneration. Hierbei wurden sämtliche Versuche mit 1g La₂O₃ 900 °C Katalysator **C**, einem molaren Verhältnis von 12 : 1 (Aceton : Citral) bei 200 °C, einem autogenen Druck von 25 bar und 3 Stunden Reaktionszeit durchgeführt.

**Tabelle 5: Recyclisierung des Katalysators ohne Regeneration.**

| Einsatz | Umsatz | Selektivität | Ausbeute |
|---|---|---|---|
| 1 | 86.8 % | 70.2 % | 60.9 % |
| 2 | 87.9 % | 75.2 % | 66.0 % |
| 3 | 79.2% | 77.4 % | 61.2% |
| 4 | 49.6 % | 77.5 % | 38.43% |
| 5 | 49.0 % | 75.4 % | 37.0 % |
| 6 | 45.5% | 74.5% | 33.9% |

### Beispiel 6

Dieses Beispiel zeigt die Recyclisierung des Katalysators mit 15-stündiger Regeneration bei 300 °C. Hierbei wurden sämtliche Versuche mit 1g La₂O₃ 900 °C Katalysator C, einem molaren Verhältnis von 12 : 1 (Aceton : Citral) bei 200 °C, einem autogenen Druck von 25 bar und 3 Stunden Reaktionszeit durchgeführt.

**Tabelle 6: Recyclisierung des Katalysators mit 15 h Regeneration bei 300 °C.**

| Einsatz | Umsatz | Selektivität | Ausbeute |
|---|---|---|---|
| 1 | 75.8% | 71.4% | 54.1 % |
| 2 | 48.9 % | 78.0 % | 38.2 % |
| 3 | 54.1 % | 79.3 % | 42.9 % |
| 4 | 31.0% | 74.2 % | 23.0 % |
| 5 | 22.6 % | 69.5 % | 15.7% |
| 6 | 27.5 % | 75.3 % | 20.7% |

### Beispiel 7:

Dieses Beispiel zeigt die Recyclisierung des Katalysators mit 15-stündiger Regeneration bei 900 °C. Hierbei wurden sämtliche Versuche mit 1g La₂O₃ 900 °C Katalysator C, einem molaren Verhältnis von 12 : 1 (Aceton : Citral) bei 200 °C, einem autogenen Druck von 25 bar und 3 Stunden Reaktionszeit durchgeführt.

**Tabelle 7: Recyclisierung des Katalysators mit 15 h Regeneration bei 900 °C.**

| Einsatz | Umsatz | Selektivität | Ausbeute |
|---|---|---|---|
| 1 | 88.7 % | 66.6 % | 59.1 % |
| 2 | 91.2% | 59.7 % | 54.4% |
| 3 | 91.2% | 66.6% | 60.8% |
| 4 | 91.2% | 59.3 % | 54.0% |
| 5 | 90.7% | 54.8% | 49.6% |
| 6 | 92.1 % | 44.2 % | 40.7 % |

Erkennbar an den Recyclisierungversuchen ist, dass eine Regeneration des Katalysators zwischen den Einsätzen zweckmässig ist. Die Regenerationstemperatur sollte dabei sehr hoch gewählt werden, da sich im Laufe der Reaktion andere Lanthanphasen bilden können, deren Ausbildung durch die Regeneration rückgängig gemacht werden kann.

### Beispiel 8

Dieses Beispiel zeigt den Einfluss des Mischungsverhältnisses Aceton : Citral auf die katalytische Aktivität. Hierbei wurden sämtliche Versuche mit 1g La₂O₃ 900 °C Katalysator **C** bei 200 °C, einem autogenen Druck von 25 bar und 3 Stunden Reaktionszeit durchgeführt.

**Tabelle 8: Einfluss des Mischungsverhältnisses auf die katalytische Aktivität.**

| Molares Mischungsverhältnis Aceton : Citral | Umsatz | Selektivität | Ausbeute |
|---|---|---|---|
| 12:1 | 66.9 % | 70.9 % | 47.4 % |
| 10 : 1 | 77.2 % | 68.7 % | 53.0 % |
| 8:1 | 71.2 % | 72.5 % | 51.6 % |
| 6:1 | 76.8% | 71.3% | 54.8% |
| 4:1 | 83.5% | 71.3% | 59.6% |
| 2:1 | 85.1 % | 64.0 % | 54.5 % |
| 1:1 | 84.3 % | 41.1 % | 34.6% |

Es konnte gezeigt werden, dass eine Verringerung des Aceton : Citral-Verhältnisses einen positiven Einfluss auf die Ausbeute hat. Sobald das Verhältnis jedoch zu niedrig wird, d. h. unter 2 : 1 sinkt, sinken Ausbeute und Selektivität stark ab, da es während der Reaktion zur Ausbildung eines zweiphasigen Systems kommt und Diffusionsprobleme auftreten.

## Patentansprüche

1. Verwendung von ungeträgertem reinem Lanthanoxid, das durch Kalzinierung von sauerstofihaltigen Lanthansalzen bei Temperaturen von mindestens 700 °C erhalten wird, als heterogener Katalysator bei der Aldolkondensation von Citral und Aceton zu Pseudojonon.

2. Verfahren zur Herstellung von Pseudojonon durch Aldolkondensation von Citral und Aceton in flüssiger Phase, **dadurch gekennzeichnet, dass** ungeträgertes reines Lanthanoxid, das durch Kalzinierung von sauerstoffhaltigen Lanthansalzen bei Temperaturen von mindestens 700 °C erhalten wird, als heterogener Katalysator verwendet wird.

## Claims

1. Use of unsupported pure lanthanum oxide which is obtained by calcination of oxygen-containing lanthanum salts at temperatures of at least 700°C as heterogeneous catalyst in the aldol condensation of citral and acetone to give pseudoionone.

2. Process for the preparation of pseudoionone by aldol condensation of citral and acetone in the liquid phase, **characterized in that** unsupported pure lanthanum oxide which is obtained by calcination of oxygen-containing lanthanum salts at temperatures of at least 700°C is used as heterogeneous catalyst.

## Revendications

1. Utilisation d'un oxyde de lanthane pur, non supporté, qui est obtenu par calcination de sels de lanthane contenant de l'oxygène à des températures d'au moins 700°C, comme catalyseur hétérogène lors de la condensation aldol de citral et d'acétone en pseudo-ionone.

2. Procédé pour la préparation de pseudo-ionone par condensation aldol de citral et d'acétone en phase liquide, **caractérisé en ce qu'**on utilise de l'oxyde de lanthane pur, non supporté, qui est obtenu par calcination de sels de lanthane contenant de l'oxygène à des températures d'au moins 700°C, comme catalyseur hétérogène.
